# EUROPEAN PATENT APPLICATION

(11) **EP 1 591 115 A1**
(43) Date of publication of application: **02.11.2005**
(21) Application number: 04703271.9
(22) Date of filing: 19.01.2004
(51) Int. Cl.: A61K 31/165, A61P 3/06, A61P 15/00, A61P 43/00

(54) **PREVENTIVE OR REMEDY FOR INTRAUTERINE LATE EMBRYONIC DEVELOPMENT OR PREGNANCY TOXEMIA**

(30) Priority: 22.01.2003 JP 2003012947
(71) Applicant: Kissei Pharmaceutical Co., Ltd., Matsumoto-shi Nagano 399-8710 (JP)
(72) Inventor: KOBAYASHI, Mamoru, Kissei Pharmaceutical Co. Ltd., Minamiazumi-gun, Nagano 399-8304 (JP); MURATA, Satoru, Kissei Pharmaceutical Co. Ltd., Minamiazumi-gun, Nagano 399-8304 (JP); TSUKAHARA, Yoshimi, Kissei Pharmaceutical Co. Ltd., Minamiazum i-gun, Nagano 399-8304 (JP)
(74) Representative: Hayes, Adrian Chetwynd
(86) International application number: PCT/JP2004/000355
(87) International publication number: WO 2004/064825

(57) **Abstract**

The present invention provides agents for the prevention or treatment of intrauterine growth retardation or gestational toxicosis comprising as an active ingredient phenylethanolaminotetralin derivatives represented by the formula (I) or pharmaceutically acceptable salts thereof such as a sulfate salt thereof, which exert a remarkable improving effect on fetal weight loss and static gangrene in the tip of fetal extremities and a remarkable improving effect on elevation of maternal urinary protein and plasma triglyceride level with ease of anxiety on palpitation of dams or the like.

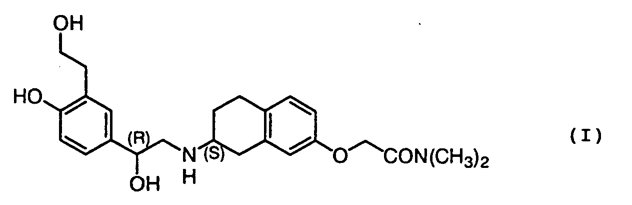

Examples of administration forms include, for example, tablets, capsules, injections and so on. As diseases to be treated, for example, asymmetrical intrauterine growth retardation and hyperlipidemia associated with gestational toxicosis are specially preferable.

## Description

### Technical Field

The present invention relates to pharmaceutical compositions which are useful for the prevention or treatment of intrauterine growth retardation or gestational toxicosis.

### Background Art

Intrauterine growth retardation (IUGR) is defined as the condition in which fetal growth is delayed or arrested and the actual fetal growth is less than the natural and latent growth corresponding to the gestation age. The condition is caused by fetal, placental and maternal factors, and these three factors coexist in most cases. Concrete causes of IUGR include fetal chromosomal aberration, intrauterine infections and abnormal metabolism, which induce the symmetrical type of IUGR in which the same degree of growth retardation is observed in the head, stature and trunk. Other concrete causes of IUGR include maternal complications such as gestational toxicosis and chronic real diseases, and disorders of the uteroplacental circulation due to placental or umbilical abnormalities, which induce the asymmetrical type of IUGR in which the growth of head and stature is within normal ranges, while that of trunk is delayed (for example, see the following Reference 1).

Gestational toxicosis, which is one of causes of IUGR, is also a risk factor to mother. Proteinuria is present, and blood fat may increase and lead to hyperlipidemia in some cases (for example, see the following References 2 and 3).

For example, bed rest, oxygen inhalation of mother and fluid therapy have been generally performed for IUGR. As drug therapies, ritodrine hydrochloride, a uterine contraction suppressor, solcoseryl, a placental function activator, theophylline and human antithrombin-III formulation have been tried. However, effectiveness of these drugs has not been established (for example, see the following References 1, 4 to 7). The basic treatment plan of gestational toxicosis includes early detection and improvement of maternal and uteroplacental circulation, and only severe cases are treated with drugs such as anti-hypertensive drugs (for example, see the following Reference 2). As described so far, there is no drug with established efficacy in treatment of IUGR and gestational toxicosis, and early development of such agents is earnestly desired.

It has been described that phenylethanolaminotetralin derivatives represented by the formula: 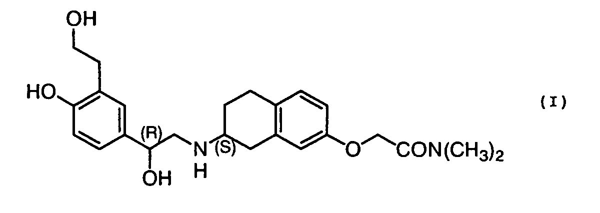 and pharmaceutically acceptable salts thereof have selective β₂-adrenoceptor stimulating activities and are useful as an agent for the prevention of threatened abortion or premature labor, a bronchodilator and an agent for pain remission or promoting stone removal in urolithiasis (see the following Reference 8). However, there has been no report on effects of the compounds on fetal weight loss, static gangrene in the tip of fetal extremities, and elevation of maternal urinary protein and plasma triglyceride level in IUGR and gestational toxicosis. It is completely unknown that the compounds are useful as agents for the prevention or treatment of IUGR or gestational toxicosis.

The purpose of the present invention is to provide an agent for the prevention or treatment of IUGR or gestation toxicosis, which improves markedly fetal weight loss, elevation of maternal urinary protein level and the like.
Reference 1: Yasushiro Kusanagi, and other 1 person, Perinatal Medicine, 2001, Vol.31, extra number, pp.328-329
Reference 2: Yukio Takasu, edited by Etsuro Ogata, [Today's therapy], 2002, published by Igaku-shoin, p.808
Reference 3: Masato Mochizuki, Perinatal Medicine, 2003, Vol.33, No.12, pp.1457-1465
Reference 4: PubMed Abstract, Gulmezoglu AM, and other 1 person, [Cochrane Database Syst. Rev.], 2001, Vol.4
Reference 5: Tetsuya Kumazawa, and other 3 persons, Acta Obstet Gynecol Jpn, 1995, Vol.47, No.9, pp.939-945
Reference 6: Cabero L. , and other 5 persons, [J. Perinat. Med.], 1998, Vol.16, No.5-6, pp.453-458
Reference 7: Japanese Patent Publication no.H8-295635
Reference 8: International Publication no.WO97/30023

### Disclosure of the Invention

The present inventors have studied earnestly to solve the above problem, and found that a phenylethanolaminotetralin derivative represented by the above formula (I) and a pharmaceutically acceptable salt thereof have a remarkable improving effect on fetal weight loss and static gangrene in the tip of fetal extremities and a remarkable improving effect on elevation of maternal urinary protein and plasma triglyceride level and that their effects on the heart are extremely weak, and therefore, they are useful as agents for the prevention or treatment of intrauterine growth retardation or gestational toxicosis, thereby forming the basis of the present invention.

That is, the present invention relates to
[1] an agent for the prevention or treatment of intrauterine growth retardation or gestational toxicosis comprising as an active ingredient a phenylethanolaminotetralin derivative represented by the formula: 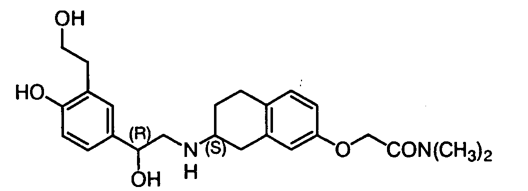 or a pharmaceutically acceptable salt thereof;
[2] an agent for the prevention or treatment as described in the above [1], wherein the active ingredient is a compound represented by the formula: 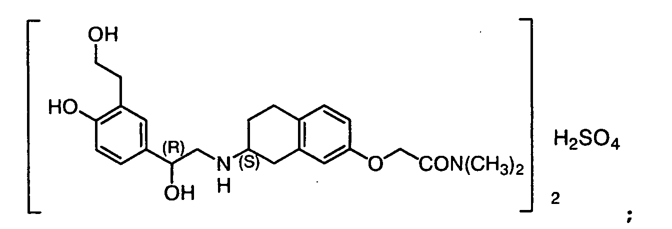
[3] an agent for the prevention or treatment as described in the above [1] or [2], wherein the disease to be treated is asymmetrical intrauterine growth retardation;
[4] an agent for the prevention or treatment as described in the above [1] or [2], wherein the disease to be treated is hyperlipidemia associated with gestational toxicosis; and the like.

More particularly, the present inventors confirmed that a compound represented by the following formula (Ia) has a surprising effect in rat intrauterine growth retardation model induced by N-nitro-L-argininemethyl ester (hereinafter referred to as L-NAME) as described below.

That is, it was confirmed that the compound exerts an excellent improving effect on fetal weight loss compared to a control group. In addition, it was also confirmed that the compound was only a slight burden to the heart at the effective dose and the anxiety on palpitation or the like was improved in dams. Thus, it has been found that the compound represented by the above formula (I) and pharmaceutically acceptable salts thereof have an excellent activity on both fetuses and dams and are extremely useful as an agent for the prevention or treatment of intrauterine growth retardation.

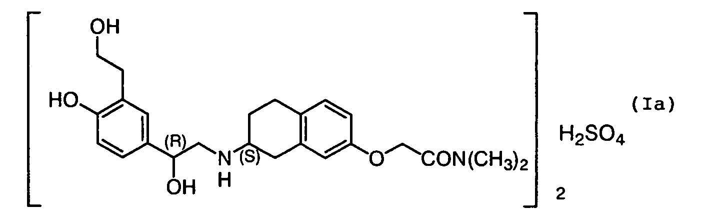

Furthermore, the present inventors confirmed that a compound represented by the following formula (Ia) has a surprisingly effect in rat L-NAME-induced gestational toxicosis model.

That is, it was confirmed that the compound exerts an excellent improving effect on static gangrene in the tip of fetal extremities compared to a control group. In addition, it was also confirmed that the compound has an excellent improving effect of the compound on elevation of maternal urinary albumin and plasma triglyceride level compared to a control group. Thus, it has been found that the compound represented by the above formula (I) and pharmaceutically acceptable salts thereof have an excellent activity on both fetuses and dams and are extremely useful as an agent for the prevention or treatment of intrauterine growth retardation.

For the reasons mentioned above, a pharmaceutical composition extremely useful as an agent for the prevention or treatment of intrauterine growth retardation or gestational toxicosis can be prepared by comprising as an active ingredient a phenylethanolaminotetralin derivative represented by the above formula (I) or a pharmaceutically acceptable salt thereof and formulating accordingly in the usual way. In the phenylethanolaminotetralin derivative of an active ingredient represented by the above formula (I), a sulphate compound represented by the above formula (Ia) is preferable. In addition, as a clinical state of the intrauterine growth retardation, asymmetric intrauterine growth retardation is particularly suitable. In gestational toxicosis, clinical states of elevation of maternal urinary protein and plasma triglyceride level are suitable, for example, hyperlipidemia associated with gestational toxicosis is particularly suitable.

A phenylethanolaminotetralin derivative represented by the above formula (I) as an active ingredient of a pharmaceutical composition of the present invention can be prepared in a method described in the above Patent Reference 1 or [Chem. Pharm. Bull.], 2001, Vol.49, No.8, pp.1018-1023, or in an analogous method or the like. In addition, as a pharmaceutically acceptable salt thereof, an acid addition salt with a mineral acid such as hydrochloric acid, hydrobromic acid, hydroiodic acid, sulfuric acid, nitric acid, phosphoric acid or the like, an acid addition salt with an organic acid such as formic acid, acetic acid, methanesulfonic acid, benzenesulfonic acid, p-toluenesulfonic acid, propionic acid, citric acid, succinic acid, tartaric acid, fumaric acid, butyric acid, oxalic acid, malonic acid, maleic acid, lactic acid, malic acid, carbonic acid, glutamic acid, aspartic acid or the like, and a salt with an inorganic base such as a sodium salt, a potassium salt or the like can be illustrated. Such a salt can be easily prepared from a phenylethanolaminotetralin derivative represented by the above formula (I) in the usual way.

When the pharmaceutical compositions of the present invention are employed in the practical treatment, they are orally or parenterally administered in various dosage forms. As examples of the orally administered dosage forms, powders, fine granules, granules, tablets, capsules, dry syrups and the like are illustrated. As examples of the parenterally administered dosage forms, injections, adhesive preparation and the like are illustrated.

These pharmaceutical compositions can be prepared by admixing with an appropriate pharmaceutical additive such as excipients, disintegrators, binders, lubricants and the like, or by heating to dissolve in an appropriate amount of purified water and optionally admixing with an appropriate pharmaceutical additive such as dissolving aids, preservatives, stabilizing agents, buffers, isotonicities and the like, and by formulating in accordance with conventional methods.

For example, tablets can be formulated by, if desired, admixing an active ingredient with appropriate diluents, disintegrating agents, binders, glidants and the like, and compressing the mixture in accordance with conventional methods. The tablets, further if desired, can be suitably coated to provide film-coated tablets, sugar-coated tablets, enteric-coated tablets and the like.

Capsules can be formulated by, if desired, admixing well an active ingredient with appropriate diluents, glidants and the like, or formulating granules or fine-powders in accordance with conventional methods, and then filling the compositions in appropriate capsules.

Injections can be formulated by heating to dissolve in an appropriate amount of purified water and, if desired, admixing with an appropriate pharmaceutical additive such as dissolving aids, preservatives, stabilizing agents, buffers, isotonicities and the like, and then dissolving completely.

The dosage of the active ingredient is appropriately decided depending on the age, body weight and degree of symptoms of each patient, which is approximately within the range of from 1 to 500 µg per day per adult human in the case of oral administration and approximately within the range of from 0.1 to 100 µg per day per adult human in the case of parenteral administration, and the daily dose can be divided into one to several doses per day and administered.

### Examples

The present invention is further illustrated in more detail by way of the following Test Examples. However, the present invention is not limited thereto.

### Test Example 1

### Confirmation of effects on IUGR

IUGR model was created as follows in accordance with the method by Yallampalli et al. (Am J Obstet Gynecol, vol.169: 1316-1320, 1993). A 17th-gestation day rat was anesthetized with ether. Then, the poll was incised approximately 10 mm to transplant in control group an osmotic pressure pump filled with L-NAME, and in the normal group, in the similar manner, an osmotic pressure pump filled with physiological saline in place of L-NAME. To the test group, in addition to the above pump, an osmotic pressure pump filled with bis(2-{[(2*S*)-2-({(2*R*)-2-hydroxy-2-[4-hydroxy-3-(2-hydroxyethyl)phenyl]ethyl}amino)-1,2,3,4-tetrahydronaphthalene-7-yl]oxy}-N,N-dimethylacetamide) monosulfate (the compound of the above formula (Ia)) was implanted. On the 20th gestation day, the mother animal was anesthetized with ether, and the fetus was extirpated and weighed. Maternal blood pressure was measured before ether anesthesia on the 17th and 20th gestation days. In statistical analysis, comparisons of control group with normal group or test group were conducted in F-test for variance. When there was equal variance, unpaired t-tests were performed. When there was unequal variance, Welch's test was performed.

Bis(2-{[(2*S*)-2-({(2*R*)-2-hydroxy-2-[4-hydroxy-3-(2-hydroxyethyl)phenyl]ethyl}amino)-1,2,3,4-tetrahydronaphthalene-7-yl]oxy}-N,N-dimethylacetamide) sulfate was dissolved in physiological saline and an osmotic pressure pump with the dose of 50 µg/day was inserted, while L-NAME was dissolved in physiological saline to make 50 mg/mL for filling and an osmotic pressure pump with the dose of 12 mg/day was inserted.

The following Table 1 shows the results of experiments concerning effects on fetal body weight. It was demonstrated that the compound represented the above formula (Ia) improved markedly the fetal weight loss associated with IUGR.

**[Table 1]**

| Group | Fetal body weight (mg) |
|---|---|
| Normal group | 3913 |
| Control group | 3400* |
| Test group | 3550 |

The mark "*" in the table indicates the significant difference of p<0.05 in comparison with the normal group.

The following Table 2 shows the results of experiments concerning effects on maternal heart. There was no change in maternal blood pressure after administration of the compound represented by the above formula (Ia), indicating the compound was only a slight burden to the heart.

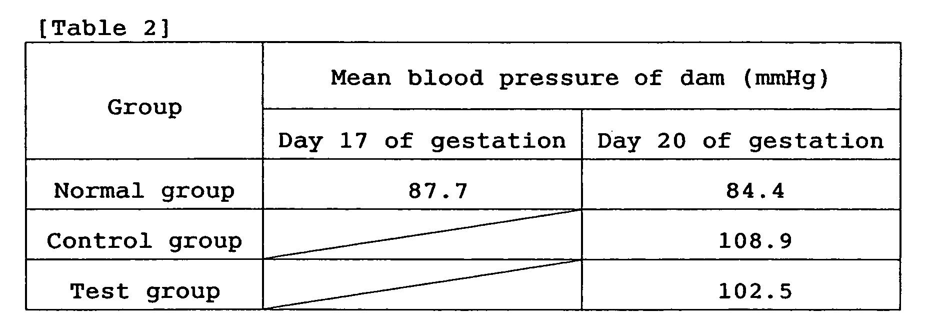

### Test Example 2

### Confirmation of effects on gestational toxicosis

A gestational toxicosis model was created as follows. A 17th -gestation day rat was anesthetized with ether. Then, the poll was incised approximately 10 mm to transplant in control group an osmotic pressure pump filled with L-NAME, and in the normal group, in the similar manner, an osmotic pressure pump filled with physiological saline in place of L-NAME. To the test group, in addition to the above pump, an osmotic pressure pump filled with bis(2-{[(2*S*)-2-({(2*R*)-2-hydroxy-2-[4-hydroxy-3-(2-hydroxyethyl)phenyl]ethyl}amino)-1,2,3,4-tetrahydronaphthalene-7-yl]oxy}-N,N-dimethylacetamide) monosulfate (the compound represented by the above formula (Ia)) was implanted. On the 20th gestation day, the mother animal was anesthetized with ether, and the fetus was extirpated. Severity of static gangrene at the tip of fetal extremities was examined, and the amount of maternal urinary albumin and plasma triglyceride level were measured.

While bis(2-{[(2*S*)-2-({(2*R*)-2-hydroxy-2-[4-hydroxy-3-(2-hydroxyethyl)phenyl]ethyl}amino)-1,2,3,4-tetrahydronaphthalene-7-yl]oxy}-N,N-dimethylacetamide) monosulfate was dissolved in physiological saline and an osmotic pressure pump with the dose of 200 µg/day was inserted, L-NAME was dissolved in physiological saline to make 200 mg/mL for filling and an osmotic pressure pump with the dose of 48 mg/day was inserted.

The following Table 3 shows the results of experiments concerning effects on static gangrene in the tip of fetal extremities. It was demonstrated that the compound represented the above formula (Ia) improved markedly said findings.

**[Table 3]**

| Group | The number of static gangrene in the tip of extremities / The number of fetuses (incidence %) |
|---|---|
| Normal group | 0/182 ( 0%) |
| Control group | 59/206* (29%) |
| Test group | 16/203# ( 8%) |

The mark "*" in the table indicates the significant difference of p<0.05 in comparison with the normal group. The mark "#" in the table indicates the significant difference of p<0.05 in comparison with the control group.

The following Table 4 shows the results of experiments concerning effects on the maternal urinary albumin level. It was demonstrated that the compound represented by the above formula (Ia) improved markedly the elevation of urinary albumin level.

**[Table 4]**

| Group | Maternal urinary albumin level(µg/mL) |
|---|---|
| Normal group | 320.5 |
| Control group | 641.7 |
| Test group | 222.1# |

The mark "#" in the table indicates the significant difference of p<0.05 in comparison with the control group.

The following Table 5 below shows results of experiments concerning effects on maternal plasma triglyceride level. It was demonstrated that the compound represented by the above formula (Ia) improved markedly the plasma triglyceride level.

**[Table 5]**

| Group | Maternal plasma triglyceride level(µg/mL) |
|---|---|
| Control group | 306.5 |
| Test group | 142.9# |

The mark "#" in the table indicates the significant difference of p<0.05 in comparison with the control group.

### Industrial Applicability

The phenylethanolaminotetralin derivatives represented by the above formula (I) or pharmaceutically acceptable salts thereof exert a remarkable improving effect on fetal weight loss and static gangrene in the tip of fetal extremities and a remarkable improving effect on elevation of maternal urinary protein and plasma triglyceride level with ease of anxiety on palpitation of dams. Therefore, pharmaceutical compositions as an active ingredient comprising the compounds are extremely useful as an agent for the prevention or treatment of intrauterine growth retardation or gestational toxicosis.

## Claims

1. An agent for the prevention or treatment of intrauterine growth retardation or gestational toxicosis, **characterized by** comprising as an active ingredient a phenylethanolaminotetralin derivative represented by the general formula: 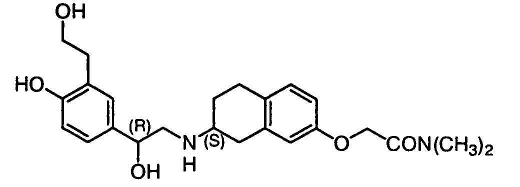 or a pharmaceutically acceptable salt thereof.

2. An agent for the prevention or treatment as claimed in claim 1, wherein the active ingredient is a compound represented by the formula:

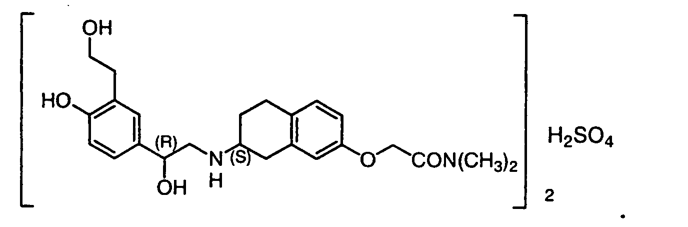

3. An agent for the prevention or treatment as claimed in claim 1 or 2, wherein the disease to be treated is asymmetrical intrauterine growth retardation.

4. An agent for the prevention or treatment as claimed in claim 1 or 2, wherein the disease to be treated is hyperlipidemia associated with gestational toxicosis.
